(19) Europäisches Patentamt
European Patent Office
Office européen des brevets

(11) Publication number: 0 413 433 A1

# (12) EUROPEAN PATENT APPLICATION

(21) Application number: 90307636.2

(51) Int. Cl.[5]: C07D 403/06, A61K 31/415

(22) Date of filing: 12.07.90

The title of the invention has been amended (Guidelines for Examination in the EPO, A-III, 7.3).

(30) Priority: 14.07.89 GB 8916150

(43) Date of publication of application: 20.02.91 Bulletin 91/08

(84) Designated Contracting States: CH DE FR GB IT LI NL

(71) Applicant: Beecham Group p.l.c.
SB House Great West Road
Brentford Middlesex TW8 9BD(GB)

(72) Inventor: Berge, John Michael, SmithKline Beecham
Pharmaceuticals, Great Burgh, Yew Tree Bottom Road
Epsom, Surrey KT18 5XQ(GB)
Inventor: Beeley, Lee James, SmithKline Beecham
Pharmaceuticals, Great Burgh, Yew Tree Bottom Road
Epsom, Surrey KT18 5XQ(GB)
Inventor: Rockell, Caroline Jeanne Margaret
415 27th Street West
Saskatoon, Saskatchewan, S7L 0J8(CA)

(74) Representative: Rutter, Keith et al
Smith Kline Beecham, Corporate Patents,
Great Burgh, Yew Tree Bottom Road
Epsom Surrey KT18 5XQ(GB)

## (54) Isoindolyl-imidazole-derivatives and pharmaceutical compositions.

(57) A compound of formula (I):

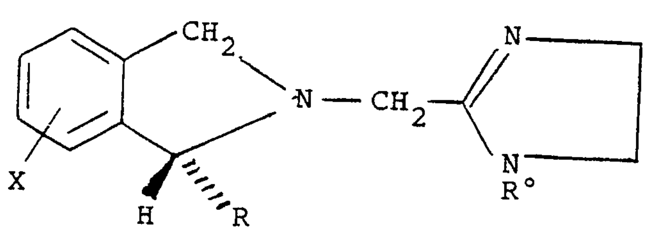

(I)

or a pharmaceutically acceptable salt thereof, or a pharmaceutically acceptable solvate thereof, wherein:
R represents an alkyl group;
R° represents a hydrogen atom, an alkyl group, a benzyl group or an alkanoyl group; and
X represents a hydrogen atom or a halogen atom; a process for preparing such a compound, a pharmaceutical composition for preparing such a compound and the use of such compounds and compositions in medicine.

EP 0 413 433 A1

# NOVEL COMPOUNDS

This invention relates to a class of heterocyclic compounds, to a process for preparing such compounds, to pharmaceutical compositions containing such compounds and to the use of such compounds and compositions in medicine.

European Patent Application, Publication Number 0,275,639 discloses compounds of formula (A):

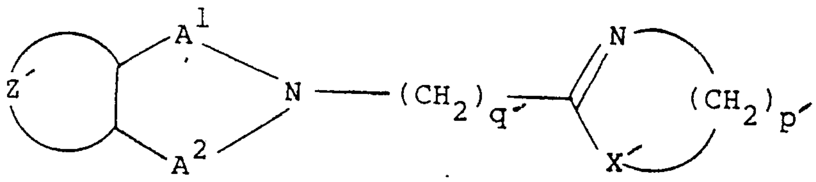

(A)

or a pharmaceutically acceptable salt, ester or amide thereof, or a pharmaceutically acceptable solvate thereof, wherein:

$Z'$ represents a residue of a substituted or unsubstituted aryl group,

$A^1$ represents a substituted or unsubstituted methylene group or a substituted or unsubstituted ethylene group;

$A^2$ represents a substituted or unsubstituted methylene group or a substituted or unsubstituted ethylene group;

providing that at least one of $A^1$ or $A^2$ represents a substituted methylene group or a substituted ethylene group,

$X'$ represents O or $NR'$ wherein $R'$ represents a hydrogen atom, a substituted or unsubstituted alkyl group, a substituted or unsubstituted aryl group, an alkanoyl group substituted or unsubstituted in the alkyl moiety, or an arylalkyl moiety substituted or unsubstituted in the aryl moiety,

$p'$ represents an integer 2 or 3, and

$q'$ represents an integer in the range of from 1 to 12.

The compounds of EP 0275639 are disclosed as having good $\alpha_2$-adrenoceptor antagonist activity and thus to be of potential use in the treatment of inter alia hyperglycaemia.

It has now been discovered that one isomeric form of the compounds of formula (A) shows advantageous $\alpha_2$-adrenoceptor antagonist activity and surprisingly is essentially free from $\alpha_1$-adrenoceptor agonist activity and hence is essentially free from the side effects associated with $\alpha_1$-adrenoceptor agonist activity. These compounds are potentially of particular value in the treatment and/or prophylaxis of hyperglycaemia and/or glaucoma and/or the treatment of hypertension and/or depression and/or for inhibiting blood platelet aggregation.

Accordingly, the present invention provides a compound of formula (I):

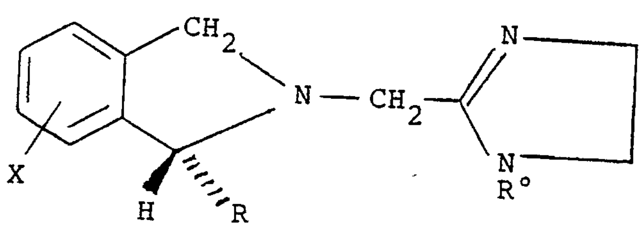

(I)

or a pharmaceutically acceptable salt thereof, or a pharmaceutically acceptable solvate thereof, wherein:

R represents an alkyl group;

$R^o$ represents a hydrogen atom, an alkyl group, a benzyl group or an alkanoyl group; and

X represents a hydrogen atom or a halogen atom.

Suitably, R represents $C_{1-6}$ alkyl.

Preferably, R represents methyl.

Suitably, $R^o$ represents hydrogen, alkyl or alkanoyl.

Preferably, $R^o$ represents hydrogen.

Preferably, X represents hydrogen.

Suitable pharmaceutically acceptable salts of the compound of formula (I) includes acid addition salts.

Suitable pharmaceutically acceptable acid addition salts of compound (I) include pharmaceutically acceptable inorganic salts such as the sulphate, nitrate, phosphate, borate, hydrochloride and hydrobromide and pharmaceutically acceptable organic acid addition salts such as acetate, tartrate, maleate, citrate, succinate, benzoate, ascorbate, methanesulphonate, α-keto glutarate, α-glycerophosphate, and glucose-1-phosphate. Preferably the acid addition salt is a hemisuccinate, hydrochloride, α-ketoglutarate, α-glycerophosphate or glucose-1-phosphate, in particular the hydrochloride salt, including the dihydrochloride.

Suitable pharmaceutically acceptable solvates includes hydrates.

When used herein the term "halogen" refers to fluorine, chlorine, bromine and iodine, favourably fluorine or chlorine.

When used herein the term "alkyl" relates to groups having straight or branched chains containing up to 12 carbon atoms.

Suitable alkyl groups are $C_{1-12}$ alkyl groups especially $C_{1-6}$ alkyl groups e.g. methyl, ethyl, n-propyl, iso-propyl, n-butyl, isobutyl or tert-butyl groups.

Suitable alkanoyl groups are $C_{1-12}$ alkanoyl groups, especially $C_{1-6}$ alkanoyl groups such as an acetyl group.

The present invention also provides a process for the preparation of a compound of formula (I), or a pharmaceutically acceptable salt thereof, or a pharmaceutically acceptable solvate thereof, which process comprises cyclising a compound of formula (II):

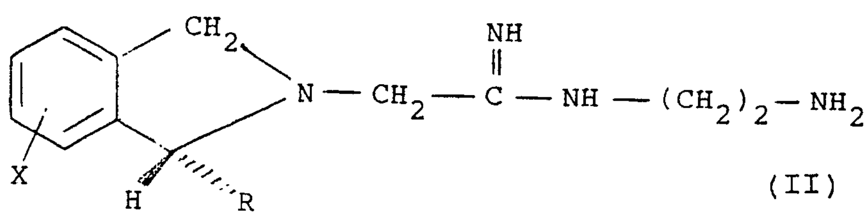

(II)

wherein X and R are as defined in relation to formula (I); and thereafter if required carrying out one or more of the following optional steps:

(i) converting a compound of formula (I) into a further compound of formula (I);

(ii) converting a compound of formula (I) into a pharmaceutically acceptable salt thereof, or a pharmaceutically acceptable solvate thereof.

A compound of formula (II) may be prepared by reacting a compound of formula (III):

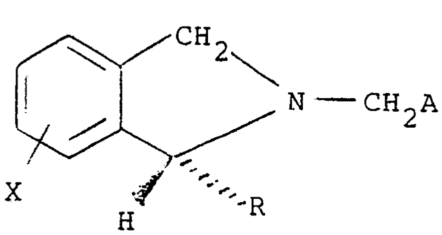

(III)

wherein X and R are as defined in relation to formula (I) and A represents -CN or $-CO_2R^2$ wherein $R^2$ represents $C_{1-6}$ alkyl, with 1,2-diaminoethane or an activated form thereof.

A suitable activated form of 1,2-diaminoethane is the trimethylaluminium adduct of 1,2-diaminoethane. An alternative activated form of 1,2-diaminoethane is provided by 1,2-diaminoethane in the presence of a

catalytic amount of carbon disulphide.

The trimethylaluminium adduct of 1,2-diaminoethane is generally the preferred reagent when A represents $-CO_2R^2$. 1,2-Diaminoethane in the presence of a catalytic amount of carbon disulphide is the preferred reagent when A represents -CN.

Suitably, $R^2$ represents methyl.

A compound of formula (III) may be prepared by reacting a compound of formula (IV):

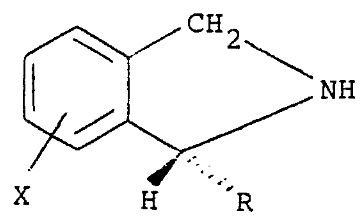

(IV)

wherein X and R are as defined in relation to formula (I), with a compound of formula (V):

$X^1-CH_2-A$ (V)

wherein A is as defined in relation to formula (III) and $X^1$ is a leaving group, and thereafter, if required, converting a compound of formula (III) into another compound of formula (III).

A suitable leaving group $X^1$ is a halogen atom such as a chlorine atom.

The reaction between the compounds of formulae (IV) and (V) may be carried out in any suitable solvent, such as acetone, at any temperature providing a suitable rate of formation of the product but conveniently at an elevated temperature, such as the boiling point of the solvent, and preferably the reaction is carried out in the presence of a base, for example potassium carbonate.

Suitable conversions of one compound of formula (III) into another compound of formula (III) include those wherein A, in formula (III), is converted from one value into another value: for example a compound of formula (III) wherein A represents nitrile may be converted into a compound of formula (III) wherein A represents $-CO_2R^2$, wherein $R^2$ is as defined in relation to formula (III), by any conventional procedure, for example by hydrolysis to give the corresponding carboxylic acid, followed by esterification.

Suitable conditions for hydrolysing, the nitrile group include acid conditions, for example using aqueous hydrobromic acid.

Suitable conditions for esterification are well known in the art and include treatment with the appropriate alcohol under acidic conditions.

A compound of formula (IV) may be prepared by reaction of a compound of formula (VI):

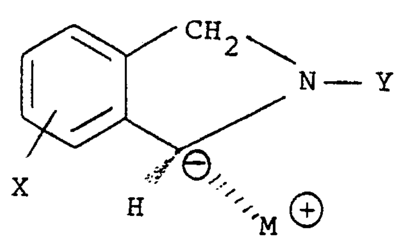

(VI)

wherein X is as defined in relation to formula (I), $M^+$ represents a metal counter-ion and Y represents a group or moiety capable of chelating with $M^+$, with a compound of formula (VII):

R-RY (VII)

wherein R is as defined in relation to formula (I) and $R^y$ represents a leaving group; and thereafter, if necessary, removing the group Y.

Suitably, $M^+$ represent an alkali metal ion, especially a lithium ion.

Suitably, Y represents a moiety of formula (a):

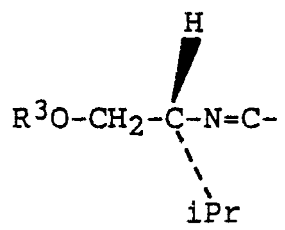

(a)

wherein $R^3$ is a $C_{1-6}$ alkyl group, preferably a tert-butyl group.

Suitably, $R^y$ represents a halogen atom, preferably an iodine atom.

Suitably, $R^3$ represents a tert-butyl group.

The reaction between the compounds of formulae (VI) and (VII) may conveniently be carried out in an aprotic solvent such as tetrahydrofuran at low to ambient temperature, but preferably at low temperature, for example at -100°C.

A compound of formula (VI) may be prepared from a compound of formula (VIII):

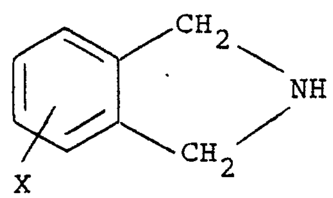

(VIII)

wherein X is as defined in relation to formula (I), with an appropriate reagent capable of forming the said group Y; and thereafter treating the compound so formed with a source of metal counter-ions, for example sec -butyllithium.

The nature of the said appropriate reagent and the required reaction conditions will of course depend upon the nature of the variable Y:

For example when Y represents a moiety of formula (a), an appropriate reagent is a compound of formula (IX):

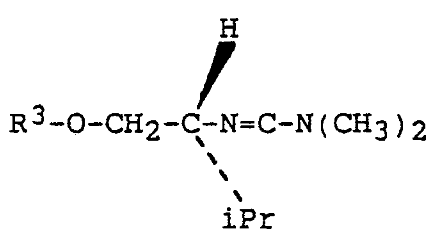

(IX)

wherein $R^3$ is as defined above.

The compounds of formula (IX) are either known compounds or they may be prepared according to methods used to prepare known compounds, for example those disclosed in the Journal of the American Chemical Society, 107 , 7974, (1985).

Suitable reaction conditions for the reaction between the compound of formula (VIII) and the appropriate reagent will of course depend upon the particular reagent used: for example when the reagent is a compound of formula (IX) the reaction may conveniently be carried out in an inert solvent, such as toluene, at any temperature providing a suitable rate of formation of the required product but generally at an elevated temperature, for example the reflux temperature of the solvent and preferably in the presence of a suitable catalyst, such as ammonium sulphate.

Where necessary, the group Y may be removed using any convenient conditions, for example when Y represents a group of formula (a) it may be removed with hydrazine hydrate and acetic acid in an aqueous

ethanolic solvent at an elevated temperature, such as in the range of from 40°C to 80°C, for example 55°C.

The compounds of formula (VIII) are known compounds or they may be prepared according to methods used to prepare known compounds for example those methods disclosed in the Journal of Organic Chemistry, 22 , 1255, (1957).

The salts and/or solvates of the compounds of formula (I) may be prepared by the appropriate conventional procedure.

The cyclisation of compounds of formula (II) may be carried out under any appropriate conditions, using any suitable solvent system and temperature range appropriate to the particular compound of formula (II), but usually at an elevated temperature.

Favourably, for the preparation of a compound of formula (I), the compound of formula (II) is not isolated from the reaction between the appropriate compound of formula (III) and 1,2-diaminoethane or an activated form thereof, thus the compound of formula (II) is converted in-situ to a compound of formula (I).

Thus, in this favoured form of the process for the preparation of compounds of formula (I), the appropriate compound of formula (III) and 1,2-diaminoethane, or more favourably an activated form thereof, are reacted together at an elevated temperature, for example within the range 80°C to 130°C, preferably 110°C, in any suitable solvent such as toluene, preferably under an atmosphere of nitrogen, or alternatively when A represents -CN the reaction may be carried out in the absence of an additional solvent.

Favourably in the last abovementioned reaction, the compound of formula (III) wherein A is $-CO_2R^2$ is reacted with the activated form of 1,2-diaminoethane, suitably the trimethylaluminium adduct of 1,2-diaminoethane.

It will be understood that under the abovementioned conditions the compound of formula (II) initially formed in the reaction between the compound of formula (III) and 1,2-diaminoethane, or an activated form thereof, subsequently undergoes cyclisation to give the required compound of formula (I).

Accordingly, in an alternative aspect the present invention provides a process for the preparation of a compound of formula (I) which process comprises reacting a compound of formula (III) with 1,2-diaminoethane, or an activated form thereof, cyclising the resulting product; and thereafter if required converting a compound of formula (I) into a pharmaceutically acceptable salt thereof.

In any of the abovementioned processes any reactive group may be protected by suitable protecting groups used conventionally in the art. The conditions for the preparation and the removal of the relevant protecting groups are those used conveniently in the art.

A compound of formula (I) may be converted into a further compound of formula (I) by using any appropriate conventional method, for example compounds wherein $R^o$ is hydrogen may be converted into a compound wherein $R^o$ is other than hydrogen by conventional alkylation, arylation, alkanoylation or aralkylation methods. Similarly compounds of formula (I) wherein $R^o$ is other than hydrogen may be converted to compounds of formula (I) wherein $R^o$ is hydrogen by conventional dealkylation, dearylation, dealkanoylation or dearylalkylation methods.

The absolute stereochemistry of any compound of formula (I), or substrate thereof, may be determined by suitable procedures, such as via conventional X-ray crystallography techniques.

The present invention also provides a compound of formula (I), or a pharmaceutically acceptable salt thereof, or a pharmaceutically acceptable solvate thereof, for use as an active therapeutic substance.

The present invention provides a compound of formula (I), or a pharmaceutically acceptable salt thereof, or a pharmaceutically acceptable solvate thereof, for use in the treatment of and/or prophylaxis of hyperglycaemia.

In a further aspect the present invention also provides a compound of formula (I) or a pharmaceutically acceptable salt thereof, or a pharmaceutically acceptable solvate thereof, for use in the treatment and/or prophylaxis of glaucoma and/or the treatment of depression and/or for inhibiting blood platelet aggregation.

In a further aspect, the present invention provides a compound of formula (I), or a pharmaceutically acceptable salt thereof, or a pharmaceutically acceptable solvate thereof, for use in the treatment of hypertension.

A compound of the (I), or a pharmaceutically acceptable salt thereof, or a pharmaceutically acceptable solvate thereof, may be administered per se or, preferably, as a pharmaceutical composition also comprising a pharmaceutically acceptable carrier.

Accordingly, the present invention also provides a pharmaceutical composition comprising a compound of formula (I), or a pharmaceutically acceptable salt thereof, or a pharmaceutically acceptable solvate thereof, and a pharmaceutically acceptable carrier therefor.

As used herein the term "pharmaceutically acceptable" embraces compounds, compositions and ingredients for both human and veterinary use: for example the term "pharmaceutically acceptable salt" embraces a veterinarily acceptable salt.

The composition may, if desired, be in the form of a pack accompanied by written or printed instructions for use.

Usually the pharmaceutical compositions of the present invention will be adapted for oral administration, although compositions for administration by other routes, such as by injection, percutaneous absorption, and, especially for the treatment and/or prophylaxis of glaucoma, topical application to the eye, are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Most suitably the composition will be formulated in unit dose form. Such unit dose will normally contain an amount of the active ingredient in the range of from 0.1 to 1000 mg, more usually 0.1 to 500 mg, and more especially 0.1 to 250 mg.

As indicated above in relation to the treatment and/or prophylaxis of glaucoma, a compound of formula (I), or a pharmaceutically acceptable salt, ester or amide thereof, or a pharmaceutically acceptable solvate thereof, may also be administered as a topical formulation in combination with conventional topical excipients. Such formulations will of course be suitably adapted for administration to the eye.

The topical formulations of the present invention may be presented as, for instance, eye ointments, creams or lotions or eye drops or other conventional formulations suitable for administration to the eye, and may contain appropriate conventional additives such as preservatives, solvents to assist drug penetration and emollients in ointments and creams. The formulations may also contain compatible conventional carriers, such as eye ointment, cream or lotion and solvents suitable for administration to the eye. Such carriers may be present as from about 20% up to about 99.5 of the formulation.

Suitable eye ointments, creams or lotions, eye drops or other conventional formulations suitable for administration to the eye are conventional formulations well known in the art, for example, as described in standard text books of pharmaceutics and cosmetics, such as Harry's Cosmeticology published by Leonard Hill Books, Remington's Pharmaceutical Sciences, and the British and US Pharmacopoeias.

Suitably, the compound of formula (I) or a pharmaceutially acceptable salt thereof, or a pharmaceutically acceptable solvate thereof, will comprise from about 0.5 to 20% by weight of the formulation, favourably from about 1 to 10% for example 2 to 5%.

The present invention further provides a method for the treatment and/or prophylaxis of hyperglycaemia in a human or non-human mammal which comprises administering an effective, non-toxic, amount of a compound of the general formula (I), or a pharmaceutically acceptable salt thereof, or a pharmaceutically acceptable solvate thereof, to a human or non-human mammal in need thereof.

The present invention further provides a method for the treatment of hypertension in a human or non-human mammal, which comprises administering an effective, non-toxic, amount of a compound of the general formula (I), or a pharmaceutically acceptable salt thereof, or a pharmaceutically acceptable solvate thereof, to a human or non-human mammal in need thereof.

The invention also provides a methcd for the treatment and/or prophylaxis of glaucoma and/or the treatment of depression and/or inhibiting blood platelet aggregation in a human or non-human mammal, which method comprises administering an effective non-toxic amount of a compound of formula (I), or a pharmaceutically acceptable salt thereof, or a pharmaceutically acceptable solvate thereof, to a human or non-human mammal in need thereof.

Conveniently, the active ingredient may be administered as a pharmaceutical composition hereinbefore defined, and this forms a particular aspect of the present invention.

In the treatment and/or prophylaxis of hyperglycaemic humans or the treatment of hypertensive humans the compound of formula (I), or a pharmaceutically acceptable salt thereof, or a pharmaceutically acceptable solvate thereof, may be taken in doses, such as those described above, one to six times a day in a manner such that the total daily dose for a 70 kg adult will generally be in the range of from 0.1 to 6000 mg, and more usually about 1 to 1500 mg.

In the treatment and/or prophylaxis of hyperglycaemic non-human mammals, especially dogs, the active ingredient may be adminstered by mouth, usually once or twice a day and in an amount in the range of from about 0.025 mg/kg to 25 mg/kg, for example 0.1 mg/kg to 20 mg/kg.

In the treatment and/or prophylaxis of glaucoma (via non-topical regimes) and the treatment of depression and inhibition of platelet aggregation in human or non-human mammals, dosage regimes are as indicated above for the treatment and/or prophylaxis of hyperglycaemic human or non-human mammals.

The present invention also provides the use of a compound of formula (I), or a pharmaceutically acceptable salt thereof, or a pharmaceutically acceptable solvate thereof, for the manufacture of a medicament for the treatment and/or prophylaxis of hyperglycaemia and/or the treatment of hypertension.

The present invention further provides the use of a compound of formula (I) or a pharmaceutically acceptable salt thereof, or a pharmaceutically acceptable solvate thereof, for the manufacture of a medicament for the treatment and/or prophylaxis of glaucoma and/or the treatment of depression and/or for the inhibition of blood platelet aggregation.

No toxicological effects are indicated when a compound of formula (I), or a pharmaceutically acceptable salt thereof, or a pharmaceutically acceptable solvate thereof, is administered in any of the abovementioned dosage ranges.

The following Examples illustrate the invention but do not limit it in any way.

Preparation 1

(R)-2-[[[1-[(1,1-Dimethylethoxy)methyl]-2-methylpropyl]imino]methyl]-2,3-dihydro-1H-isoindole

(R) 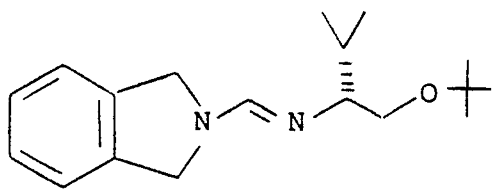

A solution of 5.38g (45.1mM) of 2,3-dihydro-1H-isoindole, 11.65g (54.3mM) of (R)-N′-( tert -butoxy-2-amino-3-methylbutyl)-N,N-dimethylformamidine and a catalytic amount of ammonium sulphate in 30ml of dry toluene, under nitrogen, was stirred at reflux temperature for 18 hours. The solution was cooled and evaporated to yield the crude product as a brown liquid.

Purification by distillation (Kugelröhr) gave the title compound as a colourless oil, bp 160°C (0.2mm/Hg).

$^1$H-nmr δ ($CDCl_3$)

7.62 (1H, s); 7.29 (4H, s); 4.70 (4H, s); 3.54 (1H, dd); 3.20 (1H, dd); 2.71 (1H, m); 1.84 (1H, m); 1.17 (9H, s); 0.89 (6H, d).

Preparation 2

(R,R)-2-[[[1-[(1,1-dimethylethoxy)methyl]-2-methyl-propyl]imino]methyl]-2,3-dihydro-1-methyl-1H-isoindole

(R,R) 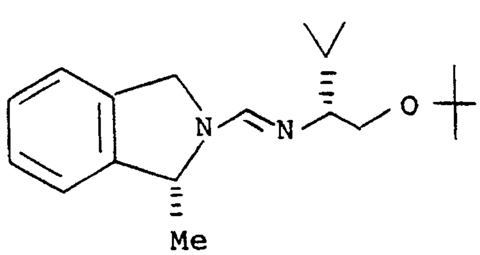

To a stirred solution of 7.0g (24.3mM) of (R)-2-[[[1-[(1,1-dimethylethoxy)methyl]-2-methylpropyl]imino]-methyl]-2,3-dihydro-1H-isoindole in 200ml of dry tetrahydrofuran, under nitrogen at -78°C, was added dropwise 20.5ml (26.7mM) of 1.3M sec -butyllithium in n-hexane. The brown solution was cooled to -100°C, then treated dropwise with 1.86ml (29.9mM) of iodomethane. After warming to -40°C over 0.5 hour the yellow solution was quenched with 20ml of water and the organic product extracted into 200ml of dichloromethane. The organic layer was washed with 50ml of water (3x), dried and evaporated to yield the crude product as a brown liquid. Purification by distillation (Kugelröhr) at 0.2mm/Hg gave the title compound as a pale red oil, bp 180-90°C.

$^1$H-nmr δ ($CDCl_3$)

7.63 (1H, s); 7.24 (4H, m); 4.98 (1H, m); 4.69 (2H, s); 3.57 (1H, dd); 3.23 (1H, t); 2.75 (1H, q); 1.87 (1H, m); 1.51 (3H, d); 1.16 (9H, s); 0.91 (3H, d); 0.89 (3H, d).
$[\alpha]D^{25}$, - 16.98° (c = 0.459, THF).

Preparation 3

(R)-2,3-Dihydro-1-methyl-1H-isoindole

(R) 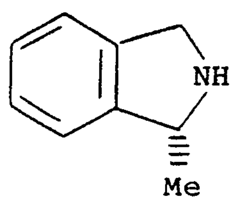

A solution of 4.12g (13.6mM) of (R,R)-2-[[[1-[(1,1-dimethylethoxy)methyl]-2-methylpropyl]imino]methyl]-2,3-dihydro-1-methyl-1H-isoindole, 2.0ml (41.1mM) of hydrazine monohydrate and 2.3ml (41.1mM) of acetic acid in 30ml of 60% (v/v) aqueous ethanol was stirred under nitrogen at 55°C for 4 hours. The reaction was cooled, evaporated and partitioned between 50ml of ethyl acetate and 20ml of saturated sodium hydrogen carbonate solution. The organic phase was separated, washed with 10ml of water and dried. Evaporation yielded the crude product as a red oil, which was chromatographed on silica gel, eluting with ethyl acetate/5% triethylamine to give the title compound as a colourless liquid.

$^1$H nmr δ ($CDCl_3$)

7.29 (4H, s); 4.44 (1H, q); 4.21 (2H, br.s); 2.18 (1H, s, exchangeable with $D_2O$), 1.40 (3H, d).

Preparation 4

(R)-1,3-Dihydro-1-methyl-2H-isoindole-2-acetonitrile

(R)
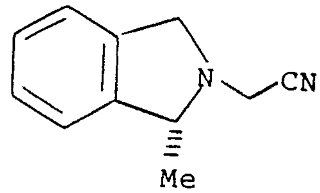

A mixture of 0.35g (2.63mM) of (R)-2,3-dihydro-1-methyl-1H-isoindole, 0.25ml (3.95mM) of chloroacetonitrile and 0.22g (1.59mM) of anhydrous potassium carbonate in 20ml of acetone was stirred under nitrogen at reflux temperature for 5 hours. The reaction was cooled, evaporated and partitioned between 15ml of dichloromethane and 5ml of 1M sodium hydrogen carbonate solution. The organic phase was separated, washed with 5ml of water and dried. Evaporation gave the crude product as a red oil, which was chromatographed on silica gel eluting with n-hexane/15% ethyl acetate to yield the title compound as a colourless oil.

$^1$H nmr δ ($CDCl_3$)

7.21 (4H, m); 4.23 (1H, d); 4.07 (2H, m); 3.85 (2H, ABq); 1.42 (3H, d).
$[\alpha]D^{25}$, -42.9° (c = 0.67, THF).

Example 1

(R)-2-[(4,5-Dihydro-1H-imidazol-2-yl)methyl]-2,3-dihydro-1-methyl-1H-isoindole

(R)-
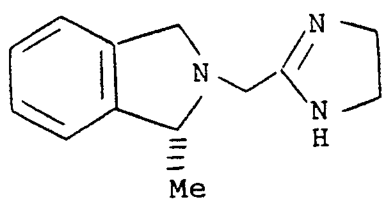

A mixture of 0.40g (2.32mM) of (R)-1,3-dihydro-1-methyl-2H-isoindole-2-acetonitrile, 0.17ml (2.54mM) of 1,2-diaminoethane and a catalytic amount of carbon disulphide was heated at 110°C under nitrogen for 1 hour. The mixture was cooled and partitioned between 10ml of dichloromethane and 5ml of water. The organic layer was washed with 5ml of water, dried and evaporated to give the crude product as red crystals. Purification by chromatography on silica gel, eluting with ethyl acetate/10% triethylamine/0-10% methanol gave the title compound as a white crystalline solid, mp 126°C (dec).

$^1$H nmr δ ($CDCl_3$)

7.19 (4H, m); 5.0 (1H, br.s, exchangeable with $D_2O$); 4.21 (1H, dd); 3.91 (1H, m); 3.72 (1H, dd); 3.63 (4H, br. s); 3.55 (2H, ABq); 1.42 (3H, d).
$[\alpha]D^{25}$, -76.2° (c = 0.38, THF).

Pharmacological Data

Post-junctional $\alpha_2$-Adrenoceptor Antagonism

To determine post-junctional $\alpha_2$-adrenoceptor activity, ring segments of rabbit lateral saphenous vein were mounted in organ baths at 37°C containing Krebs medium. Contractions of this tissue in response to noradrenaline are mediated via post-junctional $\alpha_2$-adrenoceptors (Alabaster et al , 1985) and therefore the ability of $\alpha_2$-adrenoceptor antagonists to pharmacologically antagonise such contractions gives a quantitive measure of the activity of compounds for this receptor subtype.

| Example Number | Post-synaptic $PA_2$ |
|---|---|
| 1 | 7.3 |

Alabaster V.A., Keir R.F. and Peters C.J. (1985) Naunun-Schmiedeberg's Arch Pharmacol 330, 33-36.

Post-junctional $\alpha_1$-adrenoceptor agonism

Post-junctional $\alpha_1$ activity was determined using a method similar to that for post-junctional $\alpha_2$ activity, but using rings of rabbit aorta. Contractions of this tissue are mediated by post-junctional $\alpha_1$-adrenoceptors (Docherty et al. 1981), and so the pD2 and intrinsic activity (IA) of $\alpha_1$-adrenoceptor agonists were calculated from their ability to contract the rings of aorta in comparison with noradrenaline.

| Example Number | Post-synaptic $pD_2$ |
|---|---|
| 1 | <4 |

Docherty J.R., Constantine J.W. and Starke K. 1981. Arch. Pharmocol. 317.

$\alpha$2-Adrenoceptor Binding

Human platelet membranes were incubated with [$^3$H] Rauwolscine (0.5-1.0 nM) for 30 minutes at 30°C with varying concentrates of the drug (0.1-10,000 nM). The binding assay was stopped by filtering and rinsing on GF/B glass fibre filters.

Binding Affinity (Ki) was calculated using the Cheng Prussoff equation.

| Example Number | Binding Affinity. Ki (nM) |
|---|---|
| 1 | 0.3 |

## Claims

1. A compound of formula (I) wherein:

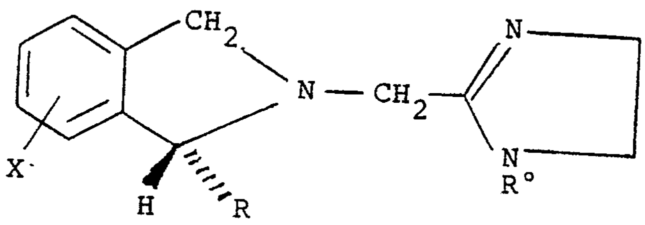

(I)

or a pharmaceutically acceptable salt thereof, or a pharmaceutically acceptable solvate thereof, characterised in that:

R represents an alkyl group;

$R^o$ represents a hydrogen atom, an alkyl group, a benzyl group or an alkanoyl group; and

X represents a hydrogen atom or a halogen atom.

2. A compound according to claim 1 wherein R represents $C_{1-6}$ alkyl, preferably methyl.

3. A compound according to claim 1 or claim 2, wherein $R^o$ represents hydrogen.

4. A compound according to any one of claims 1 to 3, wherein X represents hydrogen.

5. A compound according to claim 1 being (R)-2-[(4,5-dihydro-1H-imidazol-2-yl)methyl]-2,3-dihydro-1-methyl-1H-isoindole, or a pharmaceutically acceptable salt thereof or a pharmaceutically acceptable solvate thereof.

6. A process for the preparation of a compound of formula (I), or a pharmaceutically acceptable salt thereof, or a pharmaceutically acceptable solvate thereof, which process comprises:

a) cyclising a compound of formula (II):

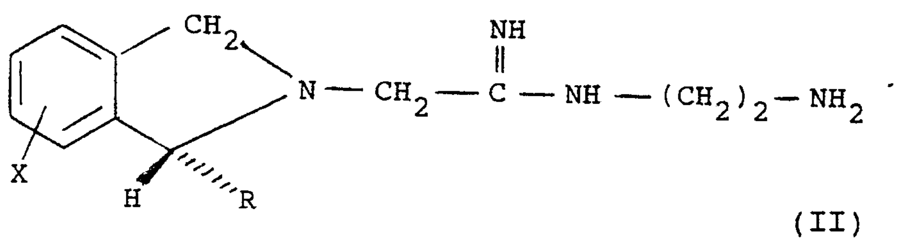

(II)

wherein X and R are as defined in relation to formula (I); or

b) reacting a compound of formula (III) with 1,2-diaminoethane, or an activated form thereof and cyclising the resulting product; and thereafter if required carrying out one or more of the following optional steps:

(i) converting a compound of formula (I) into a further compound of formula (I);

(ii) converting a compound of formula (I) into a pharmaceutically acceptable salt thereof, or a pharmaceutically acceptable solvate thereof.

7. A pharmaceutical composition comprising a compound of formula (I), or a pharmaceutically acceptable salt thereof, or a pharmaceutically acceptable solvate thereof, and a pharmaceutically acceptable carrier therefor.

8. A compound of formula (I), or a pharmaceutically acceptable salt thereof, or a pharmaceutically acceptable solvate thereof, for use as an active therapeutic substance.

9. A compound of formula (I), or a pharmaceutically acceptable salt thereof, or a pharmaceutically acceptable solvate thereof, for use in the treatment and/or prophylaxis of hyperglycaemia and/or hypertension and/or glaucoma and/or depression and/or for inhibiting blood platelet aggregation.

10. The use of a compound of formula (I), or a pharmaceutically acceptable salt thereof, or a pharmaceutically acceptable solvate thereof, for the manufacture of a medicament for the treatment and/or prophylaxis of hyperglycaemia and/or hypertension and/or glaucoma and/or depression and/or for the inhibition of blood platelet aggregation.

European Patent Office

# EUROPEAN SEARCH REPORT

Application number

EP 90307636.2

## DOCUMENTS CONSIDERED TO BE RELEVANT

| Category | Citation of document with indication, where appropriate, of relevant passages | Relevant to claim | CLASSIFICATION OF THE APPLICATION (Int Cl⁵) |
|---|---|---|---|
| P,X | EP - A1 - 0 275 639 (BEECHAM) * Claims 1,18; examples 1,5, 10 * -- | 1,7-10 | C 07 D 403/06<br>A 61 K 31/415 |
| A | EP - A1 - 0 313 288 (BEECHAM) * Claims 1,12-15 * -- | 1,7-10 | |
| A | CH - A - 526 562 (SANDOZ) * Claim 1; column 2, lines 24-34 * -- | 1,9 | |
| A | CHEMICAL ABSTRACTS, vol. 107, no. 19, November 9, 1987, Columbus, Ohio, USA BEECHAM GROUP PLC. "Preparation of new N-(heterocyclyl-alkyl)arenoheterocyclic amines for treatment of hypertension and hyerglycemia" page 726, column 2, abstract-no. 176 037j & Jpn. Kokai Tokkyo Koho JP 62,138,492 ---- | 1,9 | TECHNICAL FIELDS SEARCHED (Int Cl⁵)<br>C 07 D 403/00 |

The present search report has been drawn up for all claims

| Place of search | Date of completion of the search | Examiner |
|---|---|---|
| VIENNA | 26-09-1990 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document